# EUROPEAN PATENT APPLICATION

(11) **EP 4 563 157 A1**
(43) Date of publication of application: **04.06.2025**
(21) Application number: 23213563.2
(22) Date of filing: 30.11.2023
(51) Int. Cl.: A61K 38/17, A61P 5/00, A61P 9/00, A61P 13/12, A61P 17/00, A61P 25/28, C07K 14/00

(54) **NEW METHODS FOR TREATMENT OF NEURODEGENERATIVE DISEASES**

(71) Applicant: Vilnius University, 01513 Vilnius (LT)
(72) Inventor: Ciplys, Evaldas, Vilnius (LT); Slibinskas, Rimantas, Vilnius (LT)
(74) Representative: AAA Law

(57) **Abstract**

The invention disclosure provides compositions that inhibit amyloid protein aggregation and/or dissociate the already formed amyloid protein aggregates, both *in vitro* and *in vivo.* According to the invention disclosure, the provided compositions are useful for the treatment of neurodegenerative diseases - amyloidoses. The compositions provided herein have the advantage of using one or more chaperones of human endoplasmic reticulum (ER), selected from calnexin (CNX), BiP/GRP78, ERp57, and calreticulin (CRT). The compositions may comprise combinations of several chaperones. This description also provides method of use of such compositions, as well as pharmaceutical compositions, wherein the compositions comprise one or more of said chaperones and a pharmaceutically acceptable carrier.

## Description

### FIELD OF THE INVENTION

This invention relates to the treatment of neurodegenerative diseases, in particular, the use of endoplasmic reticulum chaperones for the treatment and prevention of neurodegenerative diseases based on the formation of amyloid plaques.

### BACKGROUND OF INVENTION

Neurodegenerative disorders (ND), such as Alzheimer's disease (AD), are one of the most important human health problems, thus it is important to address them through fundamental and applied research. There are currently no effective treatments for AD, and even more concerning is the news that clinical trials of nine new drugs that are in trials that were effective in mouse models showed no beneficial effects in patients (Holtzman, 2013). There is a 50% chance of developing AD by age of 85, and as the world's population ages, the sporadic form of AD becomes a very heavy burden on our health care resources that are limited already.

Accumulation of pathogenic chemicals of Alzheimer's disease, amyloid beta (Aβ42) peptides, leads to neurone cell death that is closely related to endoplasmic reticulum (ER) stress. Some resident chaperones of ER have been shown to translocate to different parts of the cell, including the cell surface, when ER is stressed. Endoplasmic reticulum (ER) chaperones are multifunctional proteins involved in various biological processes such as protein folding and ER quality control. The role of ER chaperones in various diseases is particularly important. The amount of native human ER chaperones is known to decline as the body ages (Holtzman, 2013). There is more and more evidence showing the involvement of certain ER chaperones in many processes. For example, the ER chaperone GRP78/BiP has been implicated in cancer progression, autoimmune inflammation and tissue damage (Panayi and Corrigall, 2006; Morito and Nagata, 2012), and rheumatoid arthritis (Corrigallet al., 2001: Yoo et al., 2012). The ER chaperone calreticulin (CRT) is important in cancer therapy (Wemeau et al., 2010) and it heals skin lesions too (Nanney et al., 2008). Other ER chaperones also exhibit protective effects, e.g., ERp57 inhibits prion neurotoxicity (Hetz et al., 2005). ER chaperones and their effects are also analysed in ND related studies (Sakono et al., 2017; Reid et al., 2022; Di Risola et al., 2022).

Current studies of the ND treatment are based on the idea that all you need to do is suppress the formation of individual toxic aggregates of specific disease-causing proteins (e.g, amyloid/amyloidogenic proteins) with some specific inhibitor and the drug will work. This way, currently only the effects are being dealt with, not the cause, so such treatments cannot have long-term positive effects. The need for substances and methods that inhibit the formation of amyloid protein aggregates and/or dissagregate/dissociate amyloid protein aggregates that are already formed, remains high.

### SUMMARY OF THE INVENTION

The invention disclosed herein provides compositions that inhibit amyloid protein aggregation and/or dissociate the already formed amyloid protein aggregates, both *in vitro* and *in vivo.* The compositions disclosed herein have the advantage of using one or more chaperones of human endoplasmic reticulum (ER), more specifically chaperones selected from calnexin (CNX), BiP/GRP78, ERp57, and calreticulin (CRT). The use of the compositions is particularly useful in inhibiting the processes of amyloid protein aggregation and/or dissociating amyloid protein aggregates that are formed already, where the amyloid proteins are amyloid beta or alpha-synuclein. This invention also reveals advantages when mixtures of several chaperones are used - in this case, a synergistic effect is observed, if compared to the use of single chaperones; it ensures complete inhibition of amyloid beta and alpha-synuclein aggregation *in vitro.* The observed effect of chaperones on amyloid proteins can be used in new compositions and methods for the treatment and prevention of ND diseases caused by the formation of amyloid plaques (fibrils). This description also discloses the method of delivering chaperones of a specific composition to the brain. It consists of an innovative method for packing proteins into exosomes and delivering prepared exosomes to the brain transnasally.

According to the first aspect, the present disclosure provides a composition for inhibiting of the aggregation of amyloidogenic proteins and/or dissociating of aggregates thereof, where the composition comprises one or more chaperones selected from: calnexin (CNX), BiP/GRP78, Erp57, and calreticulin (CRT). Amyloidogenic proteins can, for example, be selected from alpha-synuclein and amyloid beta.

According to the second aspect, the present disclosure provides a composition for inhibiting of the aggregation of amyloidogenic proteins and/or dissociating of aggregates thereof, where the composition comprises more than one chaperone selected from: calnexin (CNX), BiP/GRP78, ERp57, and calreticulin (CRT).

According to the third aspect, the present disclosure provides the use of such a composition in a method of inhibition of the aggregation of amyloid beta and/or alpha-synuclein.

According to the fourth aspect, this disclosure provides an *in vitro* method for inhibiting the aggregation of amyloid beta and/or alpha-synuclein, wherein the composition according to this invention is used.

According to the fifth aspect, this disclosure provides a pharmaceutical composition comprising the composition according to this invention in combination with a pharmaceutically acceptable carrier for use in the treatment of amyloidosis in mammals, including humans; preferably wherein the amyloidosis is Alzheimer's or Parkinson's disease.

### BRIEF DESCRIPTION OF FIGURES

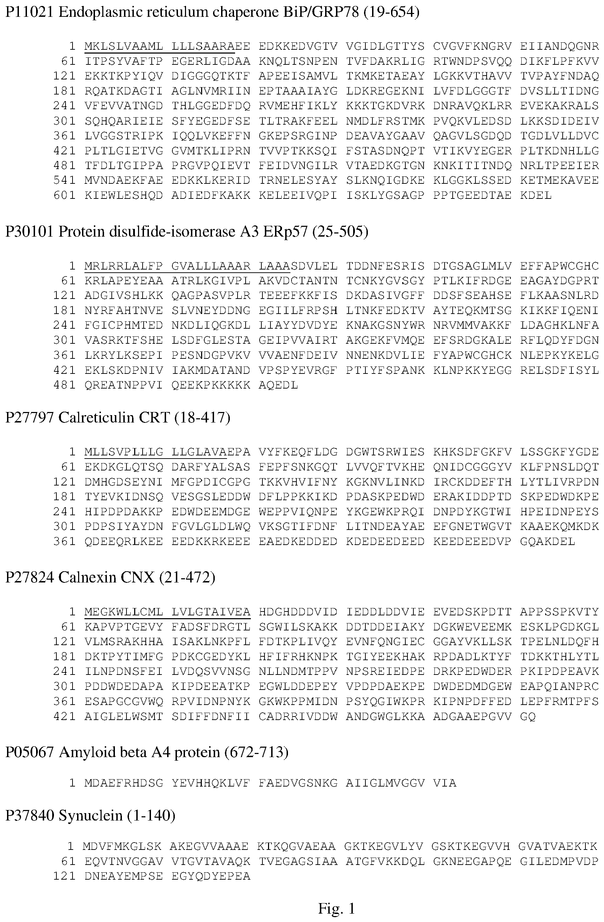

Fig. 1 Sequences of the proteins used in this description. UniProt numbers indicate the initial sequence from which the corresponding protein is taken; the figure shows the amino acid sequences (AA that are not underlined), that were used in the purified proteins that were used in the studies. The respective amino acid residues are also provided in parentheses next to the protein name. Sequences that are underlined in human chaperone sequences are signal peptides that are excised after synthesis. CNX protein with 472 AA was used, remaining 473-592 AA at the C terminus of the original protein were removed.

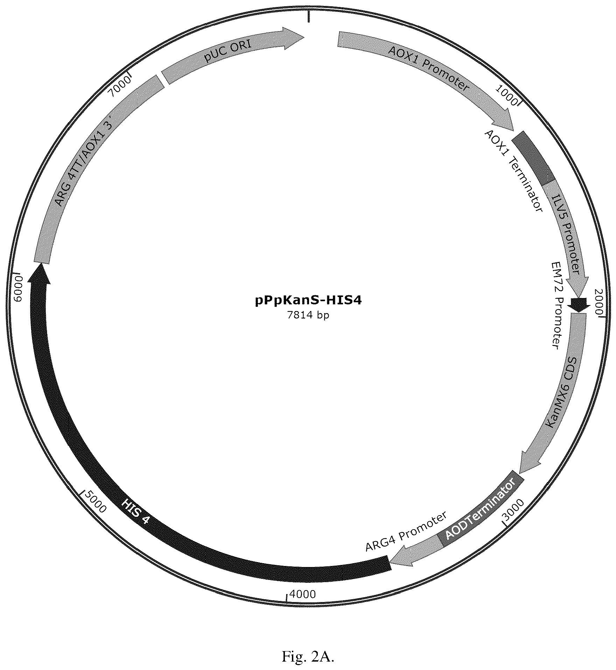

Fig. 2 A - map of plasmid pPpKanS-HIS4; B - Photo of SDS-PAGE gel. 5 ug of purified BiP, CNX, CRT, and ERp57 proteins were analyzed by SD-PAGE.

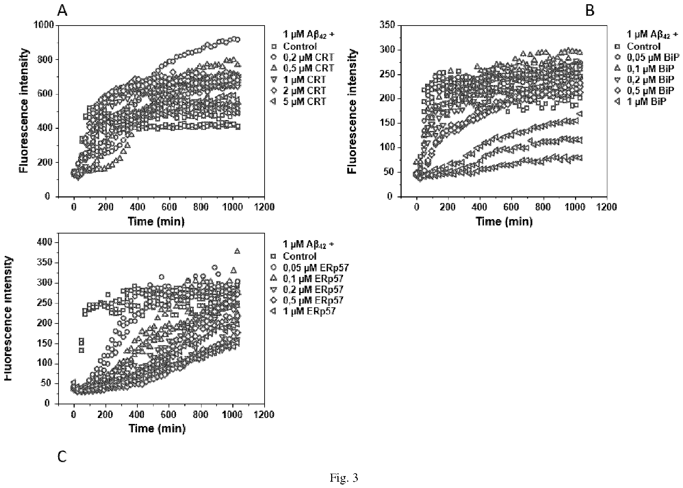

Fig. 3 Aggregation of 1 µM amyloid beta with different concentrations of CRT, BiP, and ERp57 chaperones. The fluorescent dye Thiofalcin T was used to monitor the aggregation of amyloid beta. Inhibition of aggregation is observed as a longer time required for complete aggregation of amyloid beta or as the absence of aggregation.

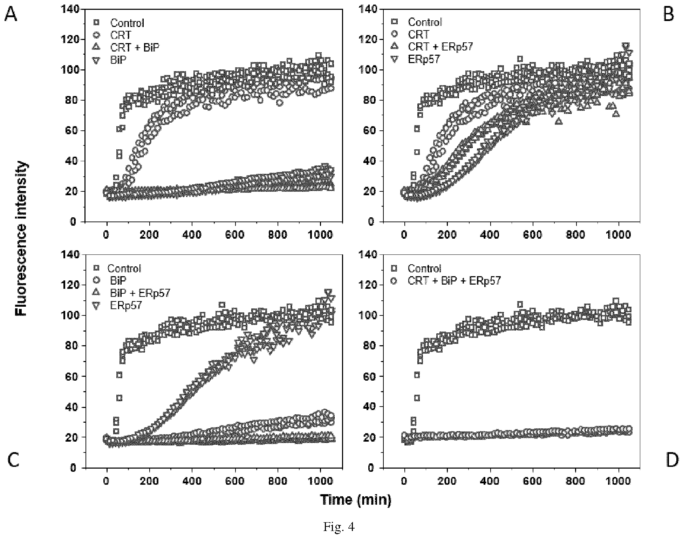

Fig. 4 Aggregation of 1 µM amyloid beta with combinations of CRT, BiP, and ERp57 chaperones. Concentrations of 1 µM CRT, 0.5 µM BiP, and 0.1 µM ERp57 were used, and fluorescent dye Thiofalcin T was used to monitor the aggregation of amyloid beta. Inhibition of aggregation is observed as a longer time required for complete aggregation of amyloid beta or as the absence of aggregation.

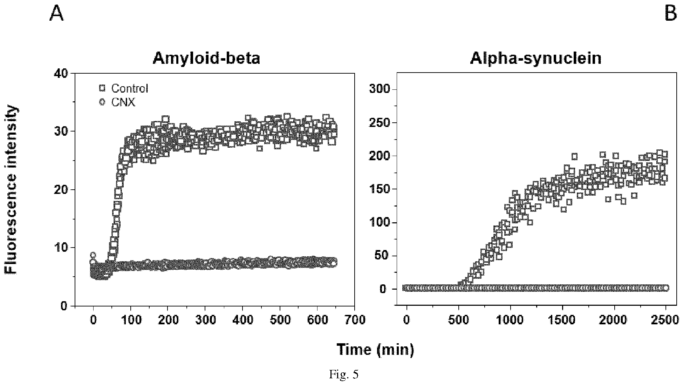

Fig. 5 Aggregation of 1 µM amyloid beta and 100 µM alpha-synuclein with the CNX chaperone. A concentration of 1 µM CNX was used. The fluorescent dye Thiofalcin T was used to monitor the aggregation of amyloid beta and alpha-synuclein. Inhibition of aggregation is observed as a longer time required for complete aggregation of amyloid beta or as the absence of aggregation.

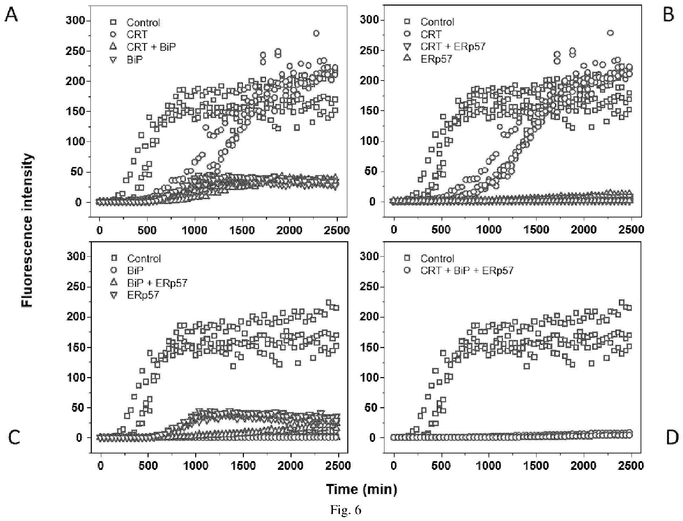

Fig. 6 Aggregation of 100 µM alpha-synuclein with CRT, BiP, and ERp57 chaperones and combinations thereof. Concentrations of 5 µM CRT, 5 µM BiP, and 5 µM ERp57 were used. The fluorescent dye Thiofalcin T was used to monitor the aggregation of alpha-synuclein. Inhibition of aggregation presents itself as a longer time required for complete aggregation of amyloid beta or as the absence of aggregation.

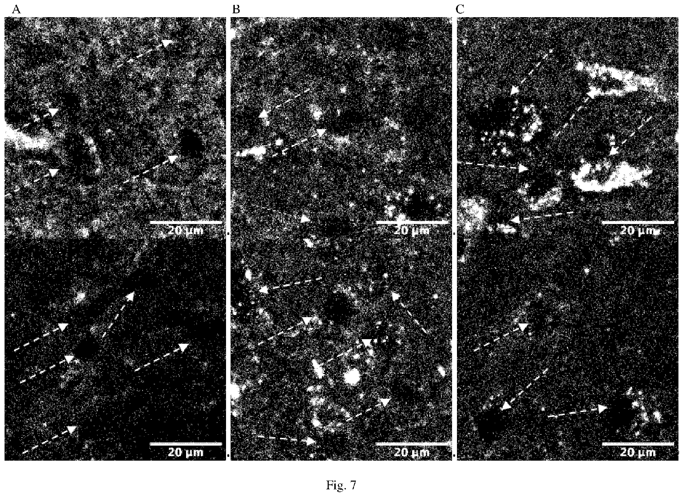

Fig. 7 Pictures of hypothalamus (top) and amygdala (bottom) brain regions of rats whose left nostril was sprayed with CRT-GFP protein-packed exosomes or PBS 4h before euthanizing them. Column A) pictures of samples from rats whose nostril was sprayed with PBS, B) - pictures of samples of the left side of the brain from rats whose nostril was sprayed with CRT-GFP-packaged exosomes, C) - samples of the right side. White dashed lines - DAPI-stained cell nuclei, white dots in rows B and C - overlay of DIL-stained exosomes (red) and CRT-GFP protein (green).

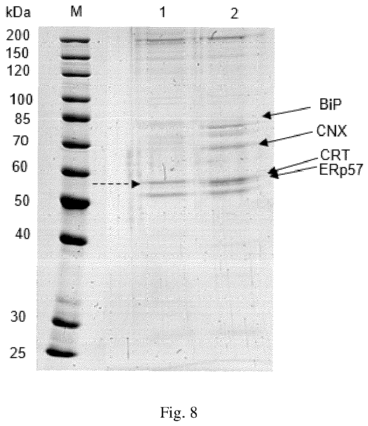

Fig. 8 SDS-PAGE analysis of samples of exosomes and exosomes with embedded proteins of endoplasmic reticulum (BiP, CNX, CRT, ERp57). M - molecular weight standard (Thermo Scientific, #26614) (5 µl), 1 - sample of exosomes isolated from mesenchymal stem cells (20 µl), 2 - sample of exosomes packaged with BiP, CNX, CRT, ERp57 proteins (20 µl).

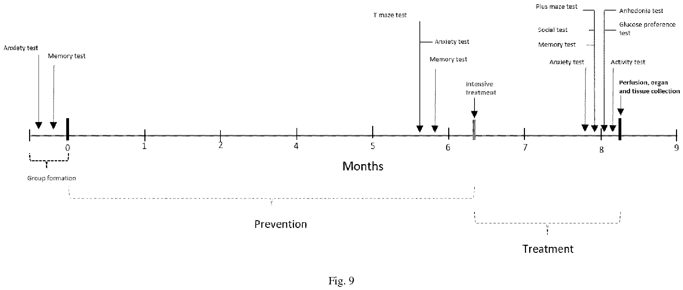

Fig. 9 Timeline of experiments performed on mice of Alzheimer's model.

### DETAILED DESCRIPTION OF THE INVENTION

This invention description provides chaperone compositions and methods of application for inhibiting the processes of formation of amyloid protein aggregates associated with neurodegenerative (ND) diseases and/or dissociating of amyloid protein aggregates that are formed already, both *in vitro* and *in vivo.*

### Amyloid/amyloidogenic proteins

Amyloid/amyloidogenic proteins are proteins capable of forming amyloid plaques/fibrils/aggregates. In this description of the invention, "amyloid fibril" refers to a fibrous supramolecular polymer formed by the aggregation of misfolded proteins (amyloid/amyloidogenic proteins) and having a structure rich in beta sheets. Examples of amyloid/amyloidogenic proteins may include proteins such as amyloid β, tau, immunoglobulin light chain, amylin, amyloid A, transthyretin, lysozyme, BriL, cystatin C, β2 microglobulin, apolipoprotein A1, fibrinogenloid, fibrinomyloid, prolactin, insulin, calcitonin, keratin, atrial natriuretic peptide, α-synuclein, prion, superoxide dismutase, neuroserpin, and α1-antichymotrypsin. Amyloid formations in human body are associated with the development of neurodegenerative diseases (amyloidosis), they disrupt the healthy function of tissues and organs.

Amyloid beta (1-42) (Aβ42) is associated with the onset and progression of Alzheimer's disease. This short peptide is believed to be the result of proteolytic processing of amyloid precursor protein (APP). It has been shown that it performs several potential physiological functions in its native form, including protection against infections, maintenance of the blood-brain barrier, and regulation of synaptic function (Brothers et al., 2018). However, its aggregates are associated with loss of synaptic signalling, generation of reactive types of oxygen and mitochondrial dysfunction, as well as neuroinflammation and loss of neurons (Cataldi et al., 2021). *In vitro* aggregation of amyloid beta (1-42) into amyloid fibrils is very rapid and spontaneous, and can occur even at room temperature, under physiological conditions without agitation (Fibers et al., 2005).

Fibrillar aggregates of alpha-synuclein (α-syn) are associated with the second most common neurodegenerative disease in humans - Parkinson's disease. This 140 amino acid presynaptic protein, which is normally found in soluble or membrane-bound fractions of the brain, aggregates into structures called Lewy bodies. Based on the relative abundance of such structures in patients with Parkinson's disease, alpha-synuclein proteins and its aggregates are indicated as one of the main causes of the disease. *In vitro,* alpha-synuclein easily forms fibrils with many different morphological and secondary structural features depending on environmental conditions and point mutations. If compared to amyloid beta, its aggregation under physiological conditions is significantly slower and requires a much higher protein concentration. (Bendor et al., 2013; Mehra et al., 2021; Giehm et al., 2010)

### Chaperones of endoplasmic reticulum (ER)

Calnexin (CNX) is an endoplasmic reticulum (ER) membrane protein with an N-terminal domain located in the ER lumen and a C-terminal domain is aimed at the cytosol. Calnexin is a molecular chaperone involved in the folding of membrane and secreted proteins.

BiP/GRP78 is a member of the ER heat shock protein 70 (Hsp70) family, an ATP-dependent chaperone. BiP can interact with both non-glycosylated and glycosylated proteins, it plays an important role in the folding of newly synthesized proteins, is also responsible for the maintenance of the ER permeability barrier during protein translocation, directing misfolded proteins for reverse translocation to be degraded by the proteasome, and has even more functions in the endoplasmic reticulum.

ERp57 (also known as PDIA3) is a member of the protein disulfide isomerase (PDI) family. ERp57 is an important mediator of the cellular response to endoplasmic reticulum stress in the disease course of several neurodegenerative disorders. Normally, ERp57 functions as a PDI and a chaperone, participates in protein folding, and interacts with calreticulin (CRT) and calnexin (CNX).

Calreticulin (CRT) is a chaperone buffering calcium of the endoplasmic reticulum. The protein is involved in the regulation of intracellular calcium homoeostasis and ER calcium capacity. Calreticulin is also involved in the folding of newly synthesized proteins and glycoproteins and, together with calnexin and ERp57, forms the "calreticulin/calnexin cycle", which is responsible for the proper folding of newly synthesized glycoproteins.

### Use of compositions comprising chaperones to inhibit the aggregation of amyloid proteins

The composition disclosed in this invention description can be used to inhibit the aggregation of amyloidogenic proteins. The composition includes one or more chaperones selected from: calnexin (CNX), BiP/GRP78, ERp57, and calreticulin (CRT). Exemplary amyloidogenic proteins whose aggregation can be inhibited by such a composition are selected from alpha-synuclein and amyloid beta. In some embodiments, the amyloidogenic protein is alpha-synuclein. In other embodiments, the amyloidogenic protein is amyloid beta.

In some embodiments, the composition comprises a CNX chaperone. In other embodiments, the composition comprises a CRT, ERp57, and/or BiP/GRP78 chaperone. A synergistic effect can be achieved when combinations or mixtures of these chaperones are used. In some examples, such as in case of the chaperone CNX, the use this single chaperone is enough to achieve the inhibition of the aggregation of amyloidogenic protein. In some other examples, CRT, ERp57, and/or BiP/GRP78 chaperones may be used individually. In other cases, it is preferable to mix several of the chaperones described herein.

When used in combination, the chaperones may be provided in the respective molar ratios described below. For example, in some embodiments, a composition is used in which BiP/GRP78, and ERp57 chaperones are provided in a molar ratio of 3:1 to 7:1, respectively; preferably the BiP/GRP78, and ERp57 chaperones are provided in a molar ratio of 5:1, respectively.

In some embodiments, a composition is used in which BiP/GRP78, and CRT chaperones are provided in a molar ratio of 1:0.5 to 1:4, respectively; preferably the BiP/GRP78, and CRT chaperones are provided in a molar ratio of 1:2, respectively.

In other embodiments, a composition is used wherein BiP/GRP78, ERp57, and CRT chaperones are provided in a molar ratio, respectively, where: BiP/GRP78 comprises 3, 4, 5, 6, or 7 parts, ERp57 comprises 1, 2, or 3 parts, and CRT comprises 7, 8, 9, 10, 11, 12, or 13 parts. Preferably, the BiP/GRP78, ERp57 and CRT chaperones are provided in a molar ratio, respectively, where BiP/GRP78 comprises 5 parts, ERp57 comprises 1 part, and CRT comprises 10 parts.

In some embodiments, a composition is used wherein ERp57 and CRT chaperones are provided in a molar ratio 1:7 and 1:13 respectively. Preferably, ERp57 and CRT chaperones are provided in a molar ratio of 1:10, respectively.

In some examples, such combinations of chaperones are used to inhibit the aggregation of amyloid beta. In yet other embodiments, a composition is used wherein BiP/GRP78, ERp57, and CRT chaperones are provided in a molar ratio, respectively, where: BiP/GRP78 comprises 1, 2, or 3 parts, ERp57 comprises 1, 2, or 3 parts, and CRT comprises 1, 2, or 3 parts. Preferably BiP/GRP78, ERp57, and CRT chaperones are provided in a 1:1:1 molar ratio, respectively. In some of these examples, such combinations of chaperones are used to inhibit alpha-synuclein aggregation.

In some embodiments, a composition is used that comprises CRT, BiP/GRP78, ERp57, and CNX chaperones.

In some embodiments, the composition has the advantage that the one or more chaperones are a native recombinant human protein obtained from a yeast expression system. During the preparation and use of chaperones in accordance with this invention disclosure of the invention, it is important that the chaperone proteins are maximally correspond to modifications of eukaryotic organisms. The technology used for the synthesis of human ER chaperones in yeast according to the principles described in WO2014011723A1 allows preparation of recombinant human ER chaperones of suitable quality so that they do not differ in their biochemical properties from native proteins.

The compositions disclosed above can be used in a method for inhibition of the aggregation of amyloid beta and/or alpha-synuclein. Such a method of inhibition of amyloid beta and/or alpha-synuclein aggregation can be *in vitro* or *in vivo* method.

In the compositions disclosed herein, the chaperones may be kept and/or stored in suitable storage buffers.

This description of the invention also provides a pharmaceutical composition for use in the treatment of amyloidosis in mammals, including humans, wherein such pharmaceutical composition comprises a composition, as dislosed according to this invention, combined with a pharmaceutically acceptable carrier. In some embodiments, the amyloidosis is Alzheimer's or Parkinson's disease. In some cases, the pharmaceutical composition, according to this invention description, comprises the CRT, BiP/GRP78, ERp57, and CNX chaperones provided in a molar ratio, respectively, where:
a) CRT comprises 7, 8, 9, 10, 11, 12, or 13 parts,
b) BiP/GRP78 comprises 1, 2, or 3 parts,
c) ERp57 comprises 1, 2, or 3 parts, and
d) CNX comprises 7, 8, 9, 10, 11, 12, or 13 parts.

In some embodiments, the CRT, BiP/GRP78, ERp57, and CNX chaperones are provided in a molar ratio of 10:5:1:10, respectively.

Thus, the chaperone compositions and combinations thereof provided herein may be useful in the treatment of amyloidoses.

### The use of compositions comprising chaperones in dissociation of amyloid protein aggregates

One of the treatments for neurodegenerative diseases characterized by amyloid formations, may be the dissociation of amyloid/amyloidogenic protein aggregates, otherwise known as aggregate dissolution or disaggregation of amyloid aggregates. Compositions described herein that comprise one or more chaperones may be used to dissociate the aggregates of amyloid/amyloidogenic proteins such as amyloid beta and alpha-synuclein. The compositions according to the present invention, disclosed in the section "Use of Compositions with Chaperones to Inhibit the Aggregation of Amyloid Proteins", may be suitable for use in dissociating amyloid protein aggregates.

### Delivery of chaperones to the brain (CNS)

In order to use the chaperones and combinations thereof, as provided in this description of the invention, for the treatment of neurodegenerative diseases such as Alzheimer's or Parkinson's disease, the chaperones need to be delivered to the brain and/or central nervous system (CNS), respectively.

Preferably the chaperones described herein may be produced in yeast, thus having the advantage of being highly similar to proteins found in human cells.

For example, the chaperones and combinations thereof disclosed in this description of the invention can be packed into exosomes, liposomes, lipid particles, microvesicles, etc. efficiently, and thus delivered to the brain. Exosomes, liposomes, and other formations may act as pharmaceutically acceptable carriers.

Chaperones and combinations thereof, according to this description of the invention, may be delivered to a target site, such as the brain, in selected therapeutically effective amounts.

For chaperones to cross the blood-brain barrier (BBB), for example, the proteins to be transferred to the brain may be modified by attaching sequences of proteins or antibodies to the proteins, so that they are recognized by receptors on the BBB and transferred to the brain (Partridge et al., 2019). Carriers, such as exosomes, can also be used. For example, the paper "Exosomes as drug delivery vehicles for Parkinson's disease therapy" (http://dx.doi.org/10.1016/j.jconrel.2015.03.033) describes the "packing" of catalase into exosomes for the treatment of Parkinson's disease. This method may be used to deliver chaperones to the cells to be treated. The chaperones of the invention may also be "packed" in a different way than described above, e.g., by lyophilizing the exosomes and resuspending them in a concentrated mixture of chaperones and separating exosomes with packed proteins from unpacked proteins by chromatography.

Chaperones and combinations thereof, according to this description of the invention, can be delivered into the body, or, for example, the body's reserves of these protective proteins can be replenished by administering them by verifiable suitable routes of administration (e.g., intravenous, subcutaneous, or other type of injections). Other methods of delivery for the treatment of neurodegenerative diseases may include oral administration of drug forms such as powders, granules, tablets, capsules, syrups, or suspensions, and parenteral administration, including injection in drug forms such as solutions, emulsions, or suspensions, as well as the form of a transdermal agent. Eye drops are recommended for the administration through the eyes, nasal drops or nasal sprays for administration through the nose, as well as oral administration. One preferred method would be trans-nasal.

The treatment or therapy described in this description of the invention can be administered to mammals. In some cases, this would be rabbits, rats, or mice. In other cases, it would be a human.

### EXAMPLES

### Example No. 1. Preparation of native recombinant products of human ER chaperones

Using the principles described in WO2014011723A1, i.e. by using the native signal sequence of the respective ER chaperone protein and synthesizing the ER chaperone with its signal sequence in yeast, four ER chaperones were synthesized and purified: calreticulin (CRT), BiP/GRP78, ERp57, and calnexin without a transmembrane domain (1-472 amino acids) (CNX). The sequences used for expression are shown in Fig. 1. The technology used for the synthesis of human ER chaperones in yeast makes it possible to prepare recombinant human ER chaperones of suitable quality. They do not differ in their biochemical properties from native proteins. Yeast vector pPpKanS-HIS4 (Fig. 2A) and stem CBS7435ΔHIS4 (Näätsaari, L et al, 2012) were used.

The required amount of calreticulin (CRT) was obtained from Baltymas PJSC.

Technologies for the production of the ER chaperones BiP, ERp57, and calnexin without a transmembrane domain (CNX) were developed, consisting of yeast *P. pastoris* cell lines and technologies of bioreactor fermentation and chromatographic purification. All stages were designed to meet the requirements of pharmaceutical proteins.

The project is based on the assumption that specifically human ER chaperones that are secreted are the main protection against all neurodegenerative diseases (ND), especially when it is known that the amount of native human ER chaperones decreases as the body ages. Therefore, our idea is to replenish the necessary reserves of these protective proteins in the body. For this purpose, it is necessary to prepare recombinant human ER chaperones of suitable quality, which do not differ in their biochemical properties from native proteins. This is made possible by the technology for the synthesis of human ER chaperones in yeast that we created and patented. It is also very important that the prepared proteins meet the requirements for pharmaceutical proteins, i.e. yeast cultivation would take place under strictly regulated conditions in a chemically defined medium, and the prepared protein preparations would be of the required purity and homogeneity, the process would be reproducible and easy to scale up.

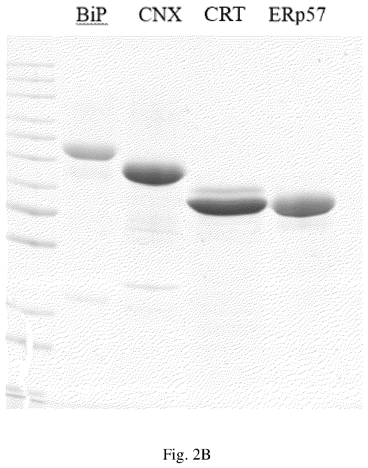

Purified homogeneous (95-98% purity according to SDS-PAGE) proteins (Fig. 2B) were sterilized by filtration and frozen for long-term storage in a suitable storage buffer.

Amyloid beta and alpha-synuclein proteins required for aggregation inhibition assays were freshly prepared before each assay as described in Sneideris et al, 2015.

### Example No. 2 Testing of CRT, BiP, ERp57, and CNX chaperones in vitro as potential inhibitors for amyloid-type fibril formation.

First, the effect of chaperones on the aggregation of the Alzheimer's disease model protein amyloid beta 1-42 (Aβ42) was tested *in vitro.* In order to evaluate the effect of chaperones on Aβ42 aggregation, it is necessary to optimize the concentration of chaperones selected for the study. If the concentration is too low, the aggregation kinetics curves would not differ from the control, while if it is too high, the reaction may take very long and will be affected by evaporation and hydroxylation of the ThT dye. A 2 µM solution of Aβ42 (20 mM sodium phosphate (pH 7.0)) was mixed with individual solutions of CRT, CNX, BiP, ERp57, or mixtures of these solutions and 10 mM amyloid dye thioflavin-T (ThT). The final concentration of Aβ42 is 1 µM; ThT - 20 µM. The optimal concentration of CRT, BiP, and ERp57 was determined by experimenting with a range of chaperone concentrations. The entire solution preparation procedure was performed on ice. The resulting reaction mixtures were dispensed into 96-well "Corning Non Binding Surface" plates, 80 µL in each well (4 units for each set of conditions). Plates were incubated at 37°C in a Clariostar Plus plate reader without shaking. Aggregation kinetics was monitored by measuring ThT fluorescence signal intensity (440 nm excitation; 480 nm emission wavelengths, measurements - every 3 minutes). It was found that when the solution contains 5 µM CRT, the reaction kinetic curves are double sigmoid (due to the possible formation of oligomers or other factors), while the reaction curves of 2 µM CRT are strongly differs from one another. Concentrations of CRT below 1 µM had no significant effect on amyloid-beta aggregation. In the case of BiP, when the solution contained 1 µM of the chaperone, the reaction kinetics curves take on an unusual shape (the reaction half-life or the duration of the lag phase cannot be determined precisely), and concentrations below 0.2 µM have no significant effect. In the case of ERp57, concentrations higher than 0.1 µM significantly slow down amyloid-beta aggregation (the curves do not reach the maximum value of the signal at the time chosen for the reaction). Based on these data, concentrations of 1 µM CRT, 0.5 µM BiP, and 0.1 µM ERp57 were chosen for further testing with amyloid-beta (Fig. 3). When the reaction mixture contained 1 µM CRT, the time required to reach the aggregation midpoint (the point in time when half of the proteins in the solution have aggregated) increased 3-fold. When the solution contained only 0.5 µM BiP, the signal intensity of Aβ42 samples increased very slowly throughout the reaction, but did not reach a maximum value. When the mixture contained 1 µM CRT and 0.5 µM BiP, no signal change was observed throughout the reaction, i.e. a complete suppression of Ab42 aggregation occurred (Fig. 4A), a synergistic effect is observed. At 0.1 µM ERp57, the reaction was inhibited more efficiently than in solution with CRT, but less so than with BiP (Fig. 3). In contrast to CRT and BiP, aggregation occurred faster with CRT and ERp57 present in the mixture than in the case of ERp57 alone (Fig. 4B). In the case of the pair of BiP and ERp57, a synergistic effect was observed - complete inhibition of aggregation, as in the case of the pair of CRT and BiP (Fig. 4C). In the presence of all three chaperones in solution (Fig. 4D), 1 µM CRT, 0.5 µM BiP, and 0.1 µM ERp57, the response was also completely suppressed. In the case of CNX, 1 µM CNX was sufficient to keep the fluorescence signal of the reaction mixture unchanged throughout the process (Fig. 5A), i.e. CNX protein alone completely inhibited aggregation of Aβ42.

The effect of all chaperones on the aggregation of alpha-synuclein (α-syn) of the Parkinson's disease model protein was tested as well. A 200 µM α-syn solution (PBS (pH 7.0)) was mixed with individual solutions of CRT, CNX, BiP, ERp57 or mixtures of these solutions and 10 mM ThT. The final α-syn concentration is 100 µM; concentration of chaperones - 5 µM each; concentration of ThT - 100 µM. Filling of assay plates with the solutions is identical to the case of Aβ42. Aggregation was observed by measuring ThT fluorescence signal intensity every 10 minutes, with continuous shaking at 600 RPM between measurements. Each well had an additional 3 mm glass bead added. CRT and BiP chaperones had similar effects on amyloid-beta aggregation (Fig. 6). In the case of CRT, the time required to reach the aggregation midpoint increased ~3-fold, while BiP strongly suppressed aggregation of α-syn (Fig. 6C). A pair of these chaperones also effectively suppressed aggregation of α-syn. In the case of ERp57, the signal intensity of the aggregation reaction was significantly lower than in the control, but the kinetic curves were of sigmoid shape. This may indicate that significantly fewer aggregates were formed, but the reaction was not completely suppressed. A mixture of CRT and ERp57 had similar effects to ERp57 alone. The mixture of BiP and ERp57 was less effective than BiP alone, but the reaction was still strongly inhibited. A mixture of all three chaperones effectively suppressed the aggregation of alpha-synuclein (Fig. 6D). CNX had the same effect as for amyloid-beta and the reaction of α-syn aggregation was completely suppressed (Fig. 5B).

During the *in vitro studies* of the inhibition of the aggregation of Alzheimer's model protein amyloid beta 1-42 (Aβ42) and Parkinson's disease model protein alpha-synuclein (α -syn) using CRT, BiP, ERp57 and CNX proteins, it was found that all tested chaperones - especially BiP and CNX - strongly inhibit the aggregation of both model proteins. During the research, the optimal concentrations and combinations of chaperones that completely inhibit the formation of model protein aggregates were identified.

### Example No. 3 Testing of CRT, BiP, ERp57, and CNX chaperones in vitro as potential factors of amyloid-type fibril dissociation.

The idea of the test is based on the fact that α-syn aggregation occurs significantly faster if 1% of already formed fibrils (the seed) is added to the α-syn monomer mixture. If the seed does not accelerate α-syn aggregation after incubation with chaperones, it means that the chaperones dissociate the seed fibrils. For this test, alpha-synuclein fibrils were prepared as a control sample for the test described above. The produced fibrils were mixed with a mixture of CRT, BiP, and ERp57 chaperones or a single CNX chaperone (concentration of all chaperones was 5 µM). These solutions were incubated at 25°C for 24 hours. Chaperone control samples were incubated without α-syn fibrils. In aggregation reactions, performed as described above, original fibrils (without incubation with chaperones), fibrils incubated with chaperones, and original fibrils freshly mixed with a mixture of CRT, BiP, and ERp57 chaperones or CNX alone were used as seeds. The final concentration of fibrils in the reaction solutions is 1 µM (according to the concentration of protein monomers), concentration of chaperones is 0.5 µM each.

CNX inhibited α-syn aggregation almost completely even with the addition of seed, which accelerates α-syn aggregation approximately 2 times.

All the obtained data suggest that human ER chaperones may protect against various neurodegenerative diseases (NDs).

### Example No. 4 Delivery of chaperones to the brain/CNS

In order for chaperones to reach the brain, it was decided to pack them in exosomes. Exosomes have been shown to effectively reach various areas of the brain via the olfactory nerve when dripped into the nose. The yeast CRT protein was synthesized and purified as a reporter protein for developing the methodology and confirming that proteins packed in exosomes enter the brain, then it was bonded with fluorescent GFP protein (CRT-GFP). Packing of proteins into exosomes requires a relatively large amount of protein, so labelling with the AlexaFlour568 dye is not always an optimal method.

First, a methodology was developed to extract exosomes from mesenchymal stem cell (MSC) growth media. The growth medium of MSC cells secreting exosomes was concentrated by tangential ultrafiltration using a VivaFlow200 (Sartorius) cartridge equipped with a PES membrane with 100 kDa pores. The size of exosomes is about 100 nm, so they, just like the proteins in the medium, are concentrated. A chromatography column with Sepharose CL-6B sorbent was used to separate exosomes from proteins and other impurities. Sepharose CL-6B allows to separate molecules by size. Purified exosomes were characterized with a Nanosight LM10 microscope - the size of exosomes (100 nm - corresponds to the theoretical size of exosomes) and the number of exosomes (about 109 units/ml) were determined.

A methodology was developed to pack the target protein into exosomes with the use of the CRT-GFP protein and purified exosomes. Several methods have been tested for packing of CRT-GFP protein into exosomes, including lyophilization, freeze-heat cycles, and sonication. CRT-GFP protein was mixed with exosomes and the mixture was treated with one of the methods indicated. After exposure, exosomes were separated from CRT-GFP protein not embedded into exosomes with the use of a Sepharose CL-6B column. Packed exosomes were analysed by SDS-PAGE and packing efficiency was evaluated based on how much of the target protein was present in the exosomes. Lyophilization proved to be the most effective, which allows for efficient packing of the target protein inside the exosomes, does not damage the structure of the exosome, and concentrates the exosomes.

200 µl CRT-GFP protein-packed exosomes marked with Dil dye were dripped into the rat's nose 20 µl every 5 min into the left side of the nose only (n=3 in each group). At 4 or 24 h after chaperone administration, rats were euthanized, perfused, and brain samples were prepared for imaging.

Summary of the results (representative images are shown in Fig. 7) allows to conclude that exosomes enter various regions of the rat brain while carrying CRT-GFP protein (Fig. 7 B and C white dots). 4h after administration, exosomes are visible both on the ipsilateral side (on the side where the drops were applied, i.e. the left) and on the contralateral side (on the side opposite to where the drops were applied, i.e. the right). Interestingly, when using an antibody against GFP, the signal intensity is significantly higher in samples obtained 24h after administration. This could be explained by the diffusion of CRT-GFP from exosomes and its better availability for reaction with the antibody. It is shown that it takes 20-25h for more than half of the protein to diffuse from exosomes. During the assessment of the localization, it seems that exosomes appear to be visible around the cells, and visualization of CRT-GFP protein with the use of an anti-GFP antibody shows CRT-GFP protein both intracellularly and in the extracellular space.

After developing an efficient method for protein delivery to the brain using exosomes, CRT, BiP/GRP78, ERp57, and CNX proteins with a molar ratio (10:5:1:10) were packed into purified exosomes using the following method:
1. 10 ml of purified exosomes were lyophilized;
2. After lyophilization, exosomes are suspended in 1 ml of a mixture of CRT, BiP/GRP78, ERp57, and CNX proteins. Kept at +4°C for 2h.
3. A Sepharose CL-6B column was used to separate protein-packed exosomes from CRT, BiP/GRP78, ERp57, and CNX proteins not embedded in exosomes while transferring the exosomes to PBS buffer at the same time.
4. Packed exosomes were analysed by SDS-PAGE and packing efficiency was evaluated based on how much target protein was present in the exosomes.
5. Purified exosomes with embedded CRT, BiP/GRP78, ERp57, and CNX proteins are frozen at -80°C.

Testing the efficiency of chaperone packaging into exosomes. It was sought to embed the human endoplasmic reticulum proteins BiP/GRP78, calreticulin (CRT), calnexin (CNX), and ERp57 into exosomes secreted by human mesenchymal cells. SDS-PAGE was performed to determine whether the target proteins were embeded in exosomes. The results of the study are presented in Figure 8. Comparison of the protein composition of exosomes and exosomes with embedded human endoplasmic reticulum proteins using the SDS-PAGE method, revealed in the latter sample additional protein bands corresponding to the electrophoretic mobility of BiP, CNX, and ERp57 proteins in the SDS-PAGE gel (Fig. 8 2, solid lines for BiP, CNX, and ERp57). The band formed by CRT protein in the NDS-PAGE gel overlaps with the exosome protein (dashed line in Fig. 8), so this band is more prominent in the sample of exosomes with embedded target proteins (Fig. 8 2, solid line CRT). After evaluating the results of SDS-PAGE analysis, it can be concluded that the human BiP, CNX, CRT, and ERp57 proteins were successfully embedded into exosomes.

*In vivo* testing. Chaperone delivery and effects are tested in the line of Alzheimer mice - B6;C3-Tg(APPswe, PSEN1dE9) 85Dbo/ Mmjax (https://www.iax.org/strain/004462).

Intranasal dripping of preparations. A total of 48 mice were divided into 2 groups: prophylactic group and treatment group.

Prophylactic group - the prophylactic group of 24 mice was divided into 3 subgroups of 8 animals, each depending on the dripped preparation: exosomes with chaperones, exosomes, and PBS. The dripping of preparations was started when the mice were 4-6 weeks old and continued to be dripped for 6 months intranasally 1 time per week. 4 µl of the respective preparation was dripped into each nostril 2 times for each mouse from each group: a total of 16 µl of preparation per mouse.

Treatment group - the treatment group of 24 mice was divided into 3 subgroups of 8 animals, each depending on the dripped preparation: exosomes with chaperones, exosomes, and PBS. The dripping of preparations was started when the mice were 28-32 weeks old and continued to be dripped for 2 months intranasally 2 times per week. The course of the procedure and the amount of the preparation are the same as in the prophylactic group. The time line of the experiments is presented in Fig. 9.

Tests of behavioural changes in mice. Employed behavioral tests: 1) Open field test, 2) Elevated Plus Maze test, 3) The Novel Object Recognition test, 4) Running Wheel, 5) Social maze, 6) Glucose preference test, 7) Sniffing test.

Murine transcardiac perfusion and organ harvesting. Perfusion - mice are anesthetized: with one hand, the mouse is secured, and an intraperitoneal infusion of anesthetic solution (a mixture of 100 µl ketamine, 50 µl xylazine, 850 µl 0.9% saline solution: 100µl/10g) is injected with the other. The mouse is returned to the cage and left for 5 minutes. After complete disappearance of reflexes, the mouse is fixed on a flat surface near the perfusion pump with its chest facing up. The chest skin and sternum are removed. The needle of the perfusion pump is inserted into the left ventricle, the heart is fixed with medical forceps. An incision is made in the right atrium with scissors. Perfusion is started. A 0.9% saline solution is infused for 3 minutes until all the blood is washed out. Organ harvesting - blood is drained into a 1.5 ml test tube as soon as perfusion is started. After perfusion, the animal is necropsied. The head is removed with scissors and the brain is extracted. The brain is split sagittally in the middle into 2 halves. Half of the brain is frozen at -80°C, the other half is placed in a tube with 4% PFA (parapharmaldehyde) solution for 24 hours for postfixation. The liver, kidneys, spleen, and cecum are also removed and frozen at -80°C in a freezer.

Preparation of brain sections, staining of brain sections. 20-µm-thick brain sections were prepared from frozen, sucrose untreated, non-paraffinized brain by cryotome sectioning under refrigeration. Staining of brain sections with Thioflavin-S. Brain sections are placed on microscope slides and left to dry overnight at room temperature. Sections are washed in 1) 50% EtOH 1 × 1 minute 2) in deionized water 1 × 3 minutes 3) Sections are incubated for 10 minutes in 0.1% Thioflavin-S. Sections are washed in 70% EtOH 2 x 3 minutes, then 50% EtOH 1 × 3 minutes, and washed in deionized water 1 × 15 minutes. Sections are dried in air for 15-30 minutes; securing medium is dripped onto the sections and a cover slip is placed. Imaging with a fluorescence scanning microscope.

### Example No. 5. Further embodiments of the present invention are presented here as a clause list:

Clause No. 1. A composition for inhibiting of the aggregation of amyloidogenic proteins and/or dissociating of aggregates thereof, wherein the composition comprises one or more chaperones selected from:
   a) calnexin (CNX),
   b) BiP/GRP78,
   c) ERp57, and
   d) calreticulin (CRT).
Clause No. 2. The composition of Clause No., wherein the composition is for inhibiting of the aggregation of amyloidogenic proteins.
Clause No. 3. The composition of Clause No. 1 or No. 2, wherein the amyloidogenic proteins are selected from alpha-synuclein and amyloid beta.
Clause No. 4. The composition of Clause No. 3, wherein the amyloidogenic protein is alpha-synuclein.
Clause No. 5. The composition of Clause No. 3, wherein the amyloidogenic protein is amyloid beta.
Clause No. 6. The composition of any of the preceding Clauses, wherein the chaperone is CNX.
Clause No. 7. The composition of any of the preceding Clauses, wherein the composition comprises a CRT, ERp57, and/or BiP/GRP78 chaperone.
Clause No. 8. The composition of Clause No. 7, wherein the composition comprises BiP/GRP78 chaperone.
Clause No. 9. The composition of Clause No. 8, wherein the composition comprises BiP/GRP78, and ERp57 chaperone.
Clause No. 10. The composition of Clause No. 8, wherein the composition comprises BiP/GRP78, and CRT chaperone.
Clause No. 11. The composition of Clause No. 9 or 10, wherein the composition comprises BiP/GRP78, ERp57, and CRT chaperone.
Clause No. 12. The composition of Clause No. 7, wherein the composition comprises ERp57 chaperone.
Clause No. 13. The composition of Clause No. 7, wherein the composition comprises CRT chaperone.
Clause No. 14. The composition of Clause No. 13, wherein the composition comprises ERp57, and CRT chaperone.
Clause No. 15. A composition of any of the preceding Clauses, wherein one or more chaperones are a native recombinant human protein obtained from a yeast expression system.
Clause No. 16. The composition of Clause No. 9, wherein the BiP/GRP78 and ERp57 chaperones are provided in a molar ratio of 3:1 to 7:1, respectively.
Clause No. 17. The composition of Clause No. 16, wherein the BiP/GRP78 and ERp57 chaperones are provided in a molar ratio of 5:1, respectively.
Clause No. 18. The composition of Clause No. 10, wherein the BiP/GRP78 and CRT chaperones are provided in a molar ratio of 1:0.5 to 1:4, respectively.
Clause No. 19. The composition of Clause No. 18, wherein the BiP/GRP78 and CRT chaperones are provided in a molar ratio of 1:2, respectively.
Clause No. 20. The composition of Clause No. 11, wherein BiP/GRP78, ERp57, and CRT chaperones are presented in a molar ratio, respectively:
   a) BiP/GRP78 comprises 3, 4, 5, 6, or 7 parts,
   b) ERp57 comprises 1, 2, or 3 parts, and
   c) CRT comprises 7, 8, 9, 10, 11, 12, or 13 parts.
Clause No. 21. The composition of Clause No. 20, wherein BiP/GRP78, ERp57 and CRT chaperones are provided in a molar ratio, where BiP/GRP78 comprises 5 parts, ERp57 comprises 1 part, and CRT comprises 10 parts.
Clause No. 22. The composition of Clause No. 4, wherein BiP/GRP78, ERp57, and CRT chaperones are provided in a molar ratio, respectively:
   a) BiP/GRP78 comprises 1, 2, or 3 parts,
   b) ERp57 comprises 1, 2, or 3 parts, and
   c) CRT comprises 1, 2, or 3 parts.
Clause No. 23. The composition of Clause No. 22, wherein BiP/GRP78, ERp57, and CRT chaperones are provided in a molar ratio 1:1:1, respectively.
Clause No. 24. The composition of Clause No. 11, wherein the ERp57 and CRT chaperones are provided in a molar ratio of 1:7 to 1:13, respectively.
Clause No. 25. The composition of Clause No. 24, wherein ERp57 and CRT chaperones are presented in a molar ratio 1:10, respectively.
Clause No. 26. Use of a composition according any of the preceding Clauses in a method of inhibition of aggregation of amyloid beta and/or alpha-synuclein.
Clause No. 27. An *in vitro* method for inhibiting the aggregation of amyloid beta and/or alpha-synuclein, wherein the composition of any of Clauses No. 1-26 is used.
Clause No. 28. A pharmaceutical composition comprising the composition of any of Clauses 1-26 in combination with a pharmaceutically acceptable carrier for use in the treatment of amyloidosis in mammals, including humans.
Clause No. 29. The pharmaceutical composition for use according to Clause No. 28, wherein the amyloidosis is Alzheimer's disease or Parkinson's disease.
Clause No. 30. The pharmaceutical composition for use according to Clause No. 28 or No. 29, wherein CRT, BiP/GRP78, ERp57, and CNX chaperones are provided in a molar ratio, respectively:
   e) CRT comprises 7, 8, 9, 10, 11, 12, or 13 parts,
   f) BiP/GRP78 comprises 1, 2, or 3 parts,
   g) ERp57 comprises 1, 2, or 3 parts, and
   h) CNX comprises 7, 8, 9, 10, 11, 12, or 13 parts.
Clause No. 31. The pharmaceutical composition for use according to Clause No. 30,wherein CRT, BiP/GRP78, ERp57, and CNX chaperones are provided in a molar ratio 10:5:1:10, respectively.
Clause No. 32. The composition of any of Clauses No. 1-5, wherein the composition comprises CRT, BiP/GRP78, ERp57, and CNX chaperones.

### References:

Bendor JT, Logan TP, Edwards RH. The function of α-synuclein. Neuron 2013;79:1044-66. https://doi.org/10.1016/j.neuron.2013.09.004.
Brothers HM, Gosztyla ML, Robinson SR. The physiological roles of amylold-β peptide hint at new ways to treat Alzheimer's disease. Front Aging Neurosci 2018;10:1-16. https://doi.org/10.3389/fnagi.2018.00118.
Cataldi R, Chia S, Pisani K, Ruggeri FS, Xu CK, Sneideris T, et al. A dopamine metabolite stabilizes neurotoxic amyloid-β oligomers. Commun Biol 2021;4:1-10. https://doi.org/10.1038/s42003-020-01490-3.
Di Risola D, Ricci D, Marrocco I, Giamogante F, Grieco M, Francioso A, Vasco-Vidal A, Mancini P, Colotti G, Mosca L, Altieri F. ERp57 chaperon protein protects neuronal cells from Aβ-induced toxicity. J Neurochem. 2022 Aug;162(4):322-336. doi: 10.1111/jnc.15655. Epub 2022 Jul 8. PMID: 35699375; PMCID: PMC9543391.
Fibers U, Nichols MR, Moss MA, Reed DK, Hoh JH, Rosenberry TL. Rapid Assembly of Amyloid-Peptide at a Liquid / Liquid Interface Produces. Society 2005:165-73.
Giehm L, Lorenzen N, Otzen DE. Assays for α-synuclein aggregation. Methods 2011;53:295-305. https://doi.org/10.1016/j.ymeth.2010.12.008.
Hetz C, Russelakis-Carneiro M, Wälchli S, Carboni S, Vial-Knecht E, Maundrell K, Castilla J, Soto C: The disulfide isomerase Grp58 is a protective factor against prion neurotoxicity. J Neurosci. 2005; 25(11):2793-802.
Holtzman DM: Cellular and animal models for high-throughput screening of therapeutic agents for the treatment of the diseases of the elderly in general and Alzheimer's disease in particular. Front Pharmacol. 2013 May 13; 4:59.
Mehra S, Gadhe L, Bera R, Sawner AS, Maji SK. Structural and functional insights into α-synuclein fibril polymorphism. Biomolecules 2021; 11. https://doi.org/10.3390/biom11101419.
Näätsaari,L., Mistlberger,B., Ruth,C., Hajek,T., Hartner,F.S., Glieder,A. (2012) Deletion of Nanney L B, Woodrell C D, Greives M R, Cardwell N L, Pollins A C, Bancroft T A, Chesser A, Michalak M, Rahman M, Siebert J W, Gold L I: Calreticulin enhances porcine wound repair by diverse biological effects. Am J PathoL 2008; 173(3):610-30.
Pardridge WM. Blood-Brain Barrier and Delivery of Protein and Gene Therapeutics to Brain. Front Aging Neurosci. 2020 Jan 10;11:373. doi: 10.3389/fnagi.2019.00373. PMID: 31998120; PMCID: PMC6966240.
Reid KM, Kitchener EJA, Butler CA, Cockram TOJ, Brown GC. Brain Cells Release Calreticulin That Attracts and Activates Microglia, and Inhibits Amyloid Beta Aggregation and Neurotoxicity. Front Immunol. 2022 Apr 20;13:859686. doi: 10.3389/fimmu.2022.859686. PMID: 35514983; PMCID: PMC9065406.
Sakono M, Kidani T. ATP-independent inhibition of amyloid beta fibrillation by the endoplasmic reticulum resident molecular chaperone GRP78. Biochem Biophys Res Commun. 2017 Nov 4;493(1):500-503. doi: 10.1016/j.bbrc.2017.08.162. Epub 2017 Sep 1. PMID: 28870813.
Sneideris T, Baranauskiene L, Cannon JG, Rutkiene R, Meškys R, Smirnovas V. Looking for a generic inhibitor of amyloid-like fibril formation among flavone derivatives. PeerJ 2015;3:e1271. https://doi.org/10.7717/peerj. 1271.
Wemeau M, Kepp 0, Tesnière A, Panaretakis T, Flament C, De Botton S, Zitvogel L, Kroemer G, Chaput N: Calreticulin exposure on malignant blasts predicts a cellular anticancer immune response in patients with acute myeloid leukemia. Cell Death Dis. 2010; 1:e104.

## Claims

1. A composition for inhibiting of the aggregation of amyloidogenic proteins and/or dissociating of aggregates thereof, wherein the composition comprises one or more chaperones selected from:
a) calnexin (CNX),
b) BiP/GRP78,
c) ERp57, and
d) calreticulin (CRT).

2. The composition of claim 1, wherein the amyloidogenic proteins are selected from alpha-synuclein and amyloid beta.

3. The composition of claim 1 or 2, wherein the chaperone is CNX.

4. The composition of claim 1 or 2, wherein the composition comprises CRT, ERp57, and/or BiP/GRP78 chaperone.

5. The composition of claim 4, wherein the composition comprises:
a) BiP/GRP78 chaperone,
b) BiP/GRP78 and ERp57 chaperone,
c) BiP/GRP78 and CRT chaperone,
d) BiP/GRP78, ERp57 and CRT chaperone,
e) ERp57 chaperone,
f) CRT chaperone,
g) ERp57 and CRT chaperone, or
h) CRT, BiP/GRP78, ERp57, and CNX chaperone.

6. The composition of any of the preceding claims, wherein the one or more chaperones is a native recombinant human protein obtained from a yeast expression system.

7. The composition of claim 5, wherein the BiP/GRP78 and ERp57 chaperones are provided in a molar ratio of 3:1 to 7:1, respectively, preferably wherein BiP/GRP78 and ERp57 chaperones are provided in a molar ratio of 5:1, respectively.

8. The composition of claim 5, wherein the BiP/GRP78 and CRT chaperones are provided in a molar ratio of 1:0.5 to 1:4, respectively, preferably wherein BiP/GRP78 and CRT chaperones are provided in a molar ratio of 1:2, respectively.

9. The composition of claim 5, wherein BiP/GRP78, ERp57, and CRT chaperones are presented in a molar ratio, respectively:
a) BiP/GRP78 comprises 3, 4, 5, 6, or 7 parts,
b) ERp57 comprises 1, 2, or 3 parts, and
c) CRT comprises 7, 8, 9, 10, 11, 12, or 13 parts,
preferably, wherein BiP/GRP78, ERp57 and CRT chaperones are provided in a molar ratio, where BiP/GRP78 comprises 5 parts, ERp57 comprises 1 part, and CRT comprises 10 parts, respectively.

10. The composition of claim 2, wherein the amyloidogenic protein is alpha-synuclein, and
wherein BiP/GRP78, ERp57 and CRT chaperones are presented in a molar ratio, respectively:
a) BiP/GRP78 comprises 1, 2, or 3 parts,
b) ERp57 comprises 1, 2, or 3 parts, and
c) CRT comprises 1, 2, or 3 parts,
preferably wherein BiP/GRP78, ERp57, and CRT chaperones are provided in a 1:1:1 molar ratio, respectively.

11. The composition of claim 5, wherein the ERp57 and CRT chaperones are provided in a molar ratio of 1:7 to 1:13, respectively, preferably wherein ERp57, and CRT chaperones are provided in a molar ratio of 1:10, respectively.

12. Use of a composition according any of the preceding claims in a method of inhibition of aggregation of amyloid beta and/or alpha-synuclein.

13. An *in vitro* method for inhibiting the aggregation of amyloid beta and/or alpha-synuclein, wherein the composition of any of claims 1-12 is used.

14. A pharmaceutical composition comprising the composition of any one of claims 1-12 in combination with a pharmaceutically acceptable carrier for use in the treatment of amyloidosis in mammals, including humans, preferably wherein the amyloidosis is Alzheimer's disease or Parkinson's disease.

15. The pharmaceutical composition for use according to claim 14, wherein CRT, BiP/GRP78, ERp57 and CNX chaperones are presented in a molar ratio, respectively:
i) CRT comprises 7, 8, 9, 10, 11, 12, or 13 parts,
j) BiP/GRP78 comprises 1, 2, or 3 parts,
k) ERp57 comprises 1, 2, or 3 parts, and
l) CNX comprises 7, 8, 9, 10, 11, 12, or 13 parts,
preferably wherein CRT, BiP/GRP78, ERp57 and CNX chaperones are provided in a 10:5:1:10 molar ratio, respectively.
